# EUROPEAN PATENT SPECIFICATION

(11) **EP 1 053 256 B1**
(45) Date of publication and mention of the grant of the patent: **20.07.2011**
(21) Application number: 99903332.7
(22) Date of filing: 25.01.1999
(51) Int. Cl.: C07K 16/28, C12N 5/20, C12N 15/13, A61K 39/395, C07K 16/46

(54) **ANTIBODIES TO DEATH RECEPTOR 4 (DR4) AND USES THEREOF**
ANTIKÖRPER GEGEN "DEATH RECEPTOR 4" (DR4) UND DEREN VERWENDUNGEN
ANTICORPS DU RECEPTEUR 4 DE LA MORT CELLULAIRE (DR4) ET LEURS UTILISATIONS

(30) Priority: 26.01.1998 US 72481 P
(43) Date of publication of application: 22.11.2000
(73) Proprietor: Genentech, Inc., South San Francisco CA 94080-4990 (US)
(72) Inventor: CHUNTHARAPAI, Anan, Colma, CA 94015 (US); KIM, Kyung, Jin, Los Altos, CA 94024 (US)
(74) Representative: Kiddle, Simon John
(86) International application number: PCT/US1999/001437
(87) International publication number: WO 1999/037684

(56) References cited:
- WO-A-92/15698
- WO-A-98/32856
- PAN G ET AL: "An antagonist decoy receptor and a death domain-containing receptor for TRAIL" SCIENCE, vol. 277, 8 August 1997, pages 815-8, XP002105803 cited in the application

## Description

### FIELD OF THE INVENTION

The present invention relates generally to DR4 antibodies, and in particular to agonist monoclonal DR4 antibodies.

### BACKGROUND OF THE INVENTION

Control of cell numbers in mammals is believed to be determined, in part, by a balance between cell proliferation and cell death. One form of cell death, sometimes referred to as necrotic cell death, is typically characterized as a pathologic form of cell death resulting from some trauma or cellular injury. In contrast, there is another, "physiologic" form of cell death which usually proceeds in an orderly or controlled manner. This orderly or controlled form of cell death is often referred to as "apoptosis" [see, *e.g.*, Barr et al., Bio/Technology, 12:487-493 (1994); Steller et al., Science,. 267:1445-1449 (1995)]. Apoptotic cell death naturally occurs in many physiological processes, including embryonic development and clonal selection in the immune system [Itoh et al., Cell, 66:233-243 (1991)]. Decreased levels of apoptotic cell death have been associated with a variety of pathological conditions, including cancer, lupus, and herpes virus infection [Thompson, Science, 267:1456-1462 (1995)]. Increased levels of apoptotic cell death may be associated with a variety of other pathological conditions, including AIDS, Alzheimer's disease, Parkinson's disease, amyotrophic lateral sclerosis, multiple sclerosis, retinitis pigmentosa, cerebellar degeneration, aplastic anemia, myocardial infarction, stroke, reperfusion injury, and toxin-induced liver disease [see, Thompson, supra].

Apoptotic cell death is typically accompanied by one or more characteristic morphological and biochemical changes in cells, such as condensation of cytoplasm, loss of plasma membrane microvilli, segmentation of the nucleus, degradation of chromosomal DNA or loss of mitochondrial function. A variety of extrinsic and intrinsic signals are believed to trigger or induce such morphological and biochemical cellular changes [Raff, Nature, 356:397-400 (1992); Steller, supra; Sachs et al., Blood, 82:15 (1993)]. For instance, they can be triggered by hormonal stimuli, such as glucocorticoid hormones for immature thymocytes, as well as withdrawal of certain growth factors [Watanabe-Fukunaga et al., Nature, 356:314-317 (1992)]. Also, some identified oncogenes such as *myc*, *rel*, and *E1A*, and tumor suppressors, like p53, have been reported to have a role in inducing apoptosis. Certain chemotherapy drugs and some forms of radiation have likewise been observed to have apoptosis-inducing activity [Thompson, supra].

Various molecules, such as tumor necrosis factor-α ("TNF-α"), tumor necrosis factor-β ("TNF-β" or "lymphotoxin-β"), lymphotoxin-β ("LT-β"), CD30 ligand, CD27 ligand, CD40 ligand, OX-40 ligand, 4-1BB ligand, Apo-1 ligand (also referred to as Fas ligand or CD95 ligand), and Apo-2 ligand (also referred to as TRAIL) have been identified as members of the tumor necrosis factor ("TNF") family of cytokines [See, e.g., Gruss and Dower, Blood, 85:3378-3404 (1995); WO 97/25428 published July 17, 1997; WO 97/01633 published January 16, 1997; Pitti et al., J. Biol. Chem., 271:12687-12690 (1996); Wiley et al., Immunity, 3:673-682 (1995); Browning et al., Cell, 72:847-856 (1993); Armitage et al. Nature, 357:80-82 (1992)]. Among these molecules, TNF-α, TNF-β, CD30 ligand, 4-1BB ligand, Apo-1 ligand, and Apo-2 ligand (TRAIL) have been reported to be involved in apoptotic cell death. Both TNF-α and TNF-β have been reported to induce apoptotic death in susceptible tumor cells [Schmid et al., Proc. Natl. Acad. Sci., 83:1881 (1986); Dealtry et al., Eur. J. Immunol., 17:689 (1987)]. Zheng et al. have reported that TNF-α is involved in post-stimulation apoptosis of CD8-positive T cells [Zheng et al., Nature, 377:348-351 (1995)]. Other investigators have reported that CD30 ligand may be involved in deletion of self-reactive T cells in the thymus [Amakawa et al., Cold Spring Harbor Laboratory Symposium on Programmed Cell Death, Abstr. No. 10, (1995)].

Mutations in the mouse Fas/Apo-1 receptor or ligand genes (called *lpr* and *gld*, respectively) have been associated with some autoimmune disorders, indicating that Apo-1 ligand may play a role in regulating the clonal deletion of self-reactive lymphocytes in the periphery [Krammer et al., Curr. Op. Immunol., 6:279-289 (1994); Nagata et al., Science, 267:1449-1456 (1995)]. Apo-1 ligand is also reported to induce post-stimulation apoptosis in CD4-positive T lymphocytes and in B lymphocytes, and may be involved in the elimination of activated lymphocytes when their function is no longer needed [Krammer et al., supra; Nagata et al., supra]. Agonist mouse monoclonal antibodies specifically binding to the Apo-1 receptor have been reported to exhibit cell killing activity that is comparable to or similar to that of TNF-α [Yonehara et al., J. Exp. Med., 169:1747-1756 (1989)].

Induction of various cellular responses mediated by such TNF family cytokines is believed to be initiated by their binding to specific cell receptors. Two distinct TNF receptors of approximately 55-kDa (TNFR1) and 75-kDa (TNFR2) have been identified [Hohman et al., J. Biol. Chem., 264:14927-14934 (1989); Brockhaus et al., Proc. Natl. Acad. Sci., 87:3127-3131 (1990); EP 417,563, published March 20, 1991] and human and mouse cDNAs corresponding to both receptor types have been isolated and characterized [Loetscher et al., Cell, 61:351 (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026 (1991)]. Extensive polymorphisms have been associated with both TNF receptor genes [see, e.g., Takao et al., Immunogenetics, 37:199-203 (1993)]. Both TNFRs share the typical structure of cell surface receptors including extracellular, transmembrane and intracellular regions. The extracellular portions of both receptors are found naturally also as soluble TNF-binding proteins [Nophar, Y. et al., EMBO J., 9:3269 (1990); and Kohno, T. et al., Proc. Natl. Acad. Sci. U.S.A., 87:8331 (1990)]. More recently, the cloning of recombinant soluble TNF receptors was reported by Hale et al. [J. Cell. Biochem. Supplement 15F, 1991, p. 113 (P424)].

The extracellular portion of type 1 and type 2 TNFRs (TNFR1 and TNFR2) contains a repetitive amino acid sequence pattern of four cysteine-rich domains (CRDs) designated 1 through 4, starting from the NH₂-terminus. Each CRD is about 40 amino acids long and contains 4 to 6 cysteine residues at positions which are well conserved [Schall et al., supra; Loetscher et al., supra; Smith et al., supra; Nophar et al., supra; Kohno et al., supra]. In TNFR1, the approximate boundaries of the four CRDs are as follows: CRD1- amino acids 14 to about 53; CRD2- amino acids from about 54 to about 97; CRD3- amino acids from about 98 to about 138; CRD4- amino acids from about 139 to about 167. In TNFR2, CRD1 includes amino acids 17 to about 54; CRD2- amino acids from about 55 to about 97; CRD3- amino acids from about 98 to about 140; and CRD4- amino acids from about 141 to about 179 [Banner et al., Cell, 73:431-435 (1993)]. The potential role of the CRDs in ligand binding is also described by Banner et al., supra.

A similar repetitive pattern of CRDs exists in several other cell-surface proteins, including the p75 nerve growth factor receptor (NGFR) [Johnson et al., Cell, 47:545 (1986); Radeke et al., Nature, 325:593 (1987)], the B cell antigen CD40 [Stamenkovic et al., EMBO J., 8:1403 (1989)], the T cell antigen OX40 [Mallet et al., EMBO J., 9:1063 (1990)] and the Fas antigen [Yonehara et al., supra and Itoh et al., Cell, 66:233-243 (1991)]. CRDs are also found in the soluble TNFR (sTNFR)-like T2 proteins of the Shope and myxoma poxviruses [Upton et al., Virology, 160:20-29 (1987); Smith et al., Biochem. Biophys. Res. Commun., 176:335 (1991); Upton et al., Virology, 184:370 (1991)]. Optimal alignment of these sequences indicates that the positions of the cysteine residues are well conserved. These receptors are sometimes collectively referred to as members of the TNF/NGF receptor superfamily. Recent studies on p75NGFR showed that the deletion of CRD1 [Welcher, A.A. et al., Proc. Natl. Acad. Sci. USA, 88:159-163 (1991)] or a 5-amino acid insertion in this domain [Yan, H. and Chao, M.V., J. Biol. Chem., 266:12099-12104 (1991)] had little or no effect on NGF binding [Yan, H. and Chao, M.V., supra]. p75 NGFR contains a proline-rich stretch of about 60 amino acids, between its CRD4 and transmembrane region, which is not involved in NGF binding [Peetre, C. et al., Eur. J. Hematol., 41:414-419 (1988); Seckinger, P. et al., J. Biol. Chem., 264:11966-11973 (1989); Yan, H. and Chao, M.V., supra]. A similar proline-rich region is found in TNFR2 but not in TNFR1.

Itoh et al. disclose that the Apo-1 receptor can signal an apoptotic cell death similar to that signaled by the 55-kDa TNFR1 [Itoh et al., supra]. Expression of the Apo-1 antigen has also been reported to be down-regulated along with that of TNFR1 when cells are treated with either TNF-α or anti-Apo-1 mouse monoclonal antibody [Krammer et al., supra; Nagata et al., supra]. Accordingly, some investigators have hypothesized that cell lines that co-express both Apo-1 and TNFR1 receptors may mediate cell killing through common signaling pathways [Id.].

The TNF family ligands identified to date, with the exception of lymphotoxin-α, are type II transmembrane proteins, whose C-terminus is extracellular. In contrast, most receptors in the TNF receptor (TNFR) family identified to date are type I transmembrane proteins. In both the TNF ligand and receptor families, however, homology identified between family members has been found mainly in the extracellular domain ("ECD"). Several of the TNF family cytokines, including TNF-α, Apo-1 ligand and CD40 ligand, are cleaved proteolytically at the cell surface; the resulting protein in each case typically forms a homotrimeric molecule that functions as a soluble cytokine. TNF receptor family proteins are also usually cleaved proteolytically to release soluble receptor ECDs that can function as inhibitors of the cognate cytokines.

Recently, other members of the TNFR family have been identified. Such newly identified members of the TNFR family include CAR1, HVEM and osteoprotegerin (OPG) [Brojatsch et al., Cell, 87:845-855 (1996); Montgomery et al., Cell, 87:427-436 (1996); Marsters et al., J. Biol. Chem., 272:14029-14032 (1997); Simonet et al., Cell, 89:309-319 (1997)]. Unlike other known TNFR-like molecules, Simonet et al., supra, report that OPG contains no hydrophobic transmembrane-spanning sequence.

In Marsters et al., Curr. Biol., 6:750 (1996), investigators describe a full length native sequence human polypeptide, called Apo-3, which exhibits similarity to the TNFR family in its extracellular cysteine-rich repeats and resembles TNFR1 and CD95 in that it contains a cytoplasmic death domain sequence [see also Marsters et al., Curr. Biol., 6:1669 (1996)]. Apo-3 has also been referred to by other investigators as DR3, wsl-1 and TRAMP [Chinnaiyan et al., Science, 274:990 (1996); Kitson et al., Nature, 384:372 (1996); Bodmer et al., Immunity, 6:79 (1997)].

Pan et al. have disclosed another TNF receptor family member referred to as "DR4" [Pan et al., Science, 276:111-113 (1997)]. The DR4 cDNA encodes an open reading frame of 468 amino acids with features characteristic of a cell surface receptor. Pan et al. describe a putative signal peptide present at the beginning of the molecule (amino acids -23 to -1), with the mature protein predicted to start at amino acid 24 (Ala). Residues 108 to 206 contain two cysteine-rich pseudorepeats that resemble corresponding regions in TNFR-1 (four repeats), DR3 (four repeats), Fas (three repeats) and CAR1 (two repeats). Following the transmembrane domain is an intracellular region containing a 70 amino acid stretch with similarity to the death domains of TNFR1, DR3, Fas, and CAR1. The DR4 transcript was detected in spleen, peripheral blood leukocytes, small intestine, and thymus. In addition, DR4 expression was also found in K562 erythroleukemia cells, MCF7 breast carcinoma cells and activated T cells. Pan et al. further disclose that DR4 is believed to be a receptor for the ligand known as Apo-2 ligand or TRAIL.

In Sheridan et al., Science, 277:818-821 (1997) and Pan et al., Science, 277:815-818 (1997), another molecule believed to be a receptor for the Apo-2 ligand (TRAIL) is described. That molecule is referred to as Apo-2 (it has also been alternatively referred to as DR5). Like DR4, Apo-2 is reported to contain a cytoplasmic death domain and be capable of signaling apoptosis.

In Sheridan et al., supra, a receptor called DcR1 (or alternatively, Apo-2DcR) is disclosed as being a potential decoy receptor for Apo-2 ligand (TRAIL). Sheridan et al. report that DcR1 can inhibit Apo-2 ligand function *in vitro*. See also, Pan et al., supra, for disclosure on the decoy receptor referred to as TRID.

In Marsters et al., Curr. Biol., 7:1003-1006 (1997), a receptor referred to as DcR2 is disclosed. Marsters et al. report that DcR2 contains a cytoplasmic region with a truncated death domain and can function as an inhibitory Apo-2L receptor *in vitro*.

For a review of the TNF family of cytokines and their receptors, see Gruss and Dower, supra.

As presently understood, the cell death program contains at least three important elements - activators, inhibitors, and effectors; in *C. elegans*, these elements are encoded respectively by three genes, *Ced-4*, *Ced-9* and *Ced-3* [Steller, Science, 267:1445 (1995); Chinnaiyan et al., Science, 275:1122-1126 (1997); Wang et al., Cell, 90:1-20 (1997)]. Two of the TNFR family members, TNFR1 and Fas/Apol (CD95), can activate apoptotic cell death [Chinnaiyan and Dixit, Current Biology, 6:555-562 (1996); Fraser and Evan, Cell; 85:781-784 (1996)]. TNFR1 is also known to mediate activation of the transcription factor, NF-KB [Tartaglia et al., Cell, 74:845-853 (1993); Hsu et al., Cell, 84:299-308 (1996)]. In addition to some ECD homology, these two receptors share homology in their intracellular domain (ICD) in an oligomerization interface known as the death domain [Tartaglia et al., supra; Nagata, Cell, 88:355 (1997)]. Death domains are also found in several metazoan proteins that regulate apoptosis, namely, the Drosophila protein, Reaper, and the mammalian proteins referred to as FADD/MORT1, TRADD, and RIP [Cleaveland and Ihle, Cell, 81:479-482 (1995)]. Upon ligand binding and receptor clustering, TNFR1 and CD95 are believed to recruit FADD into a death-inducing signaling complex. CD95 purportedly binds FADD directly, while TNFR1 binds FADD indirectly via TRADD [Chinnaiyan et al., Cell, 81:505-512 (1995); Boldin et al., J. Biol. Chem., 270:387-391 (1995); Hsu et al., supra; Chinnaiyan et al., J. Biol, Chem., 271:4961-4965 (1996)]. It has been reported that FADD serves as an adaptor protein which recruits the Ced-3-related protease, MACHα/FLICE (caspase 8), into the death signaling complex [Boldin et al., Cell, 85:803-815 (1996); Muzio et al., Cell, 85:817-827 (1996)]. MACHα/FLICE appears to be the trigger that sets off a cascade of apoptotic proteases, including the interleukin-1β converting enzyme (ICE) and CPP32/Yama, which may execute some critical aspects of the cell death program [Fraser and Evan, supra].

It was recently disclosed that programmed cell death involves the activity of members of a family of cysteine proteases related to the *C. elegans* cell death gene, *ced-3*, and to the mammalian IL-1-converting enzyme, ICE. The activity of the ICE and CPP32/Yama proteases can be inhibited by the product of the cowpox virus gene, crmA [Ray et al., Cell, 69:597-604 (1992); Tewari et al., Cell. 81:801-809 (1995)]. Recent studies show that CrmA can inhibit TNFR1- and CD95-induced cell death [Enari et al., Nature, 375:78-81 (1995); Tewari et al., J. Biol. Chem., 270:3255-3260 (1995)].

As reviewed recently by Tewari et al., TNFR1, TNFR2 and CD40 modulate the expression of proinflammatory and costimulatory cytokines, cytokine receptors, and cell adhesion molecules through activation of the transcription factor, NF-KB [Tewari et al., Curr. Op. Genet. Develop., 6:39-44 (1996)]. NF-KB is the prototype of a family of dimeric transcription factors whose subunits contain conserved Rel regions [Verma et al., Genes Develop., 9:2723-2735 (1996); Baldwin, Ann. Rev. Immunol., 14:649-681 (1996)]. In its latent form, NF-KB is complexed with members of the IKB inhibitor family; upon inactivation of the IKB in response to certain stimuli, released NF-KB translocates to the nucleus where it binds to specific DNA sequences and activates gene transcription. WO 98/32856, citable under Art. 54(3) EPC, relates to DR4 polypeptide.

### SUMMARY OF THE INVENTION

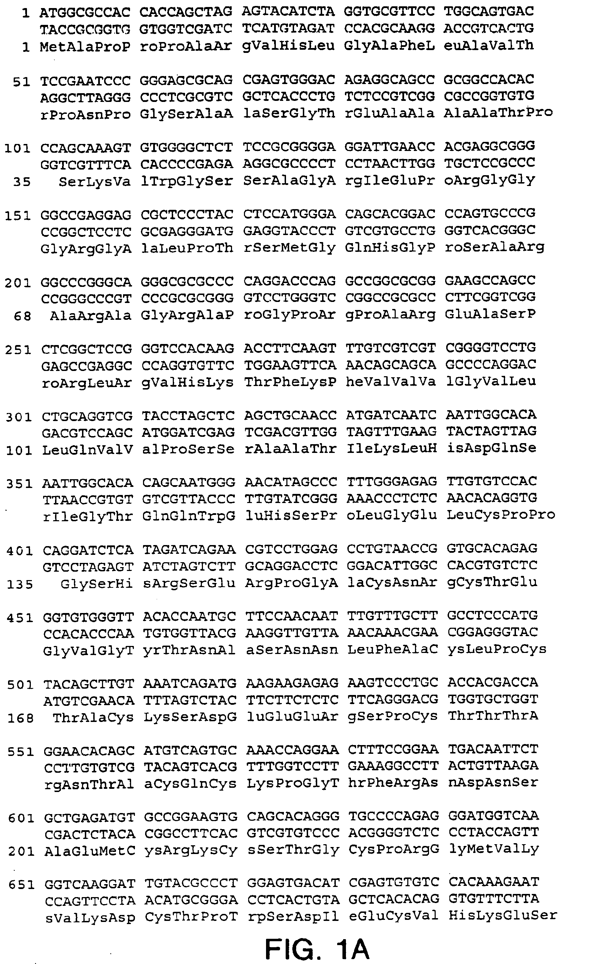

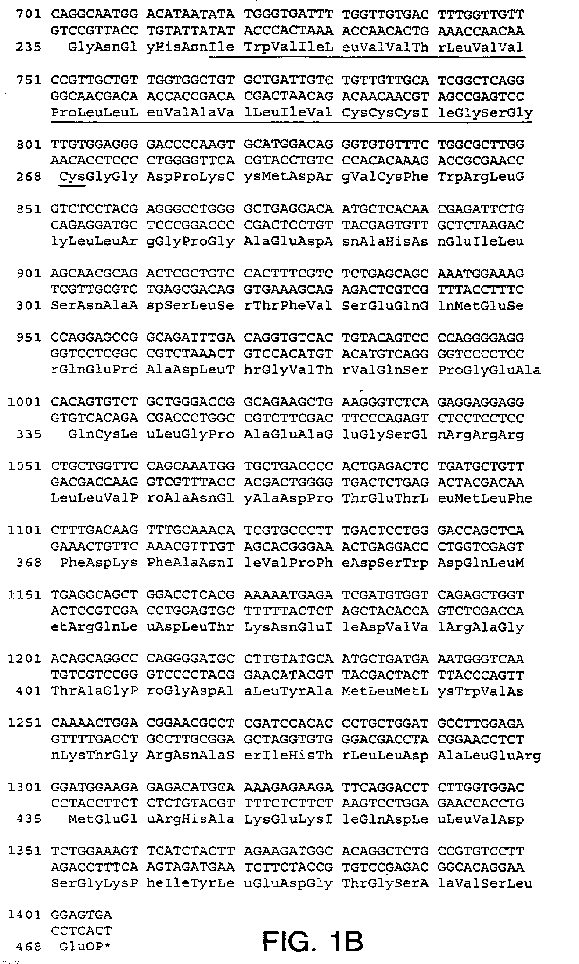

The present invention relates to isolated agonist monoclonal antibody which (a) specifically binds to a DR4 polypeptide comprising amino acid residues 24-218, 1-218, or 1-468 of Fig. 1 (SEQ ID NO:1), (b) induces apoptosis in at least one type of mammalian cell that expresses DR4 polypeptide and (c) does not bind to DcR1 or DcR2, and thus are useful in the treatment of various diseases and pathological conditions, including cancer.

The invention also provides hybridoma cell lines which produce DR4 monoclonal antibodies.

The invention also provides compositions comprising one or more DR4 antibodies and a carrier, such as a pharmaceutically-acceptable carrier. In one embodiment, such composition may be included in an article of manufacture or kit.

### BRIEF DESCRIPTION OF THE DRAWINGS

Figure 1 shows the nucleotide sequence (SEQ ID NO:2) of a cDNA for human DR4 and its derived amino acid sequence (SEQ ID NO:1). The respective nucleotide and amino acid sequences for human DR4 are also reported in Pan et al., Science, 276:111 (1997).
Figures 2 shows the FACS analysis of two anti-DR4 antibodies, 4E7.24.3 and 4H6.17.8 (illustrated by the bold lines) as compared to IgG controls (dotted lines). Both antibodies recognized the DR4 receptor expressed in human 9D cells.
Figure 3 is a graph showing percent (%) apoptosis induced in 9D cells by DR4 antibodies, 4E7.24.3 and 4H6.17.8, in the absence of goat anti-mouse IgG Fc.
Figure 4 is a bar diagram showing percent (%) apoptosis, as compared to Apo-2L, in 9D cells by DR4 antibodies, 4E7.24.3 and 4H6.17.8, in the presence or absence of goat anti-mouse IgG Fc.
Figure 5 is a bar diagram illustrating the ability of DR4 antibody 4H6.17.8 to block the apoptosis induced by Apo-2L in 9D cells.
Figure 6 is a graph showing results of an ELISA testing binding of DR4 antibodies, 4E7.24.3 and 4H6.17.8, to DR4 and to other known Apo-2L receptors referred to as Apo-2, DcR1, and DcR2.

### DETAILED DESCRIPTION OF THE PREFERRED EMBODIMENTS

### I. Definitions

As used herein, the term "Apo-2 ligand" or "Apo-2L" (also known as TRAIL) refers to a specific member of the tumor necrosis factor (TNF) ligand family that induces apoptosis in a variety of cell lineages [see WO 97/25428 published July 17,1997; Pitti et al., J. Biol. Chem, 271:12687 (1996); Marsters et al., Curr. Biol., 6:79 (1997); Wiley, S. et al., Immunity, 3:637 (1995)].

A receptor for Apo-2L has been identified and referred to as DR4, a member of the TNF-receptor family that contains a cytoplasmic "death domain" capable of engaging the cell suicide apparatus [see Pan et al., Science, 276:111 (1997)]. The term "Death Receptor 4" or "DR4" when used herein encompasses native sequence DR4 and DR4 variants (which are further defined herein). These terms encompass DR4 expressed in a variety of mammals, including humans. DR4 may be endogenously expressed as occurs naturally in a variety of human tissue lineages, or may be expressed by recombinant or synthetic methods. A "native sequence DR4" comprises a polypeptide having the same amino acid sequence as a DR4 derived from nature. Thus, a native sequence DR4 can have the amino acid sequence of naturally-occurring DR4 from any mammal. Such native sequence DR4 can be isolated from nature or can be produced by recombinant or synthetic means. The term "native sequence DR4" specifically encompasses naturally-occurring truncated or secreted forms of the DR4 (*e.g.*, a soluble form containing, for instance, an extracellular domain sequence), naturally-occurring variant forms (*e.g.*, alternatively spliced forms) and naturally-occurring allelic variants of the DR4. In one embodiment of the invention, the native sequence DR4 is a mature or full-length native sequence DR4 comprising amino acids 1 to 468 of Fig. 1 (SEQ ID NO:1).

The terms "extracellular domain" or "ECD" herein refer to a form of DR4 which is essentially free of the transmembrane and cytoplasmic domains of DR4. Ordinarily, DR4 ECD will have less than 1% of such transmembrane and/or cytoplasmic domains and preferably, will have less than 0.5% of such domains. Optionally, DR4 ECD will comprise amino acid residues 1 to 218 or residues 24 to 218 of Fig. 1 (SEQ ID NO:1).

"DR4 variant" means a biologically active DR4 having at least about 80% or 85% amino acid sequence identity with the DR4 having the deduced amino acid sequence shown in Fig. 1 (SEQ ID NO:1) for a full-length native sequence human DR4. Such DR4 variants include, for instance, DR4 polypeptides wherein one or more amino acid residues are added, or deleted, at the N- or C-terminus of the sequence of Fig. 1 (SEQ ID NO:1). Ordinarily, an DR4 variant will have at least about 80% amino acid sequence identity, more preferably at least about 90% amino acid sequence identity, and even more preferably at least about 95% amino acid sequence identity with the amino acid sequence of Fig. 1 (SEQ ID NO:1).

"Percent (%) amino acid sequence identity" with respect to the DR4 sequences identified herein is defined as the percentage of amino acid residues in a candidate sequence that are identical with the amino acid residues in the DR4 sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as ALIGN^{™} or Megalign (DNASTAR) software. Those skilled in the art can determine appropriate parameters for measuring alignment, including any algorithms needed to achieve maximal alignment over the full length of the sequences being compared.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the DR4 natural environment will not be present. Ordinarily, however, isolated polypeptide will be prepared by at least one purification step.

The terms "agonist" and "agonistic" when used herein refer to or describe a molecule which is capable of, directly or indirectly, substantially inducing, promoting or enhancing DR4 biological activity or activation.

The terms "antagonist" and "antagonistic" when used herein refer to or describe a molecule which is capable of, directly or indirectly, substantially counteracting, reducing or inhibiting DR4 biological activity or DR4 activation.

The term "antibody" is used in the broadest sense and specifically covers single anti-DR4 monoclonal antibodies (including agonist, antagonist, and neutralizing or blocking antibodies) and anti-DR4 antibody compositions with polyepitopic specificity. "Antibody" as used herein includes intact immunoglobulin or antibody molecules, polyclonal antibodies, multispecific antibodies (i.e., bispecific antibodies formed from at least two intact antibodies) and immunoglobulin fragments (such as Fab, F(ab')₂, or Fv), so long as they exhibit any of the desired agonistic properties described herein.

Antibodies are typically proteins or polypeptides which exhibit binding specificity to a specific antigen. Native antibodies are usually heterotetrameric glycoproteins, composed of two identical light (L) chains and two identical heavy (H) chains. Typically, each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H)} followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains [Chothia et al., J. Mol. Biol., 186:651-663 (1985); Novotny and Haber, Proc. Natl. Acad. Sci. USA, 82:4592-4596 (1985)]. The light chains of antibodies from any vertebrate species can be assigned to one of two clearly distinct types, called kappa (6) and lambda (8), based on the amino acid sequences of their constant domains. Depending on the amino acid sequence of the constant domain of their heavy chains, immunoglobulins can be assigned to different classes. There are five major classes of immunoglobulins: IgA, IgD, IgE, IgG and IgM, and several of these may be further divided into subclasses (isotypes), e.g., IgG-1, IgG-2, IgG-3, and IgG-4; IgA-1 and IgA-2. The heavy chain constant domains that correspond to the different classes of immunoglobulins are called alpha, delta, epsilon, gamma, and mu, respectively.

"Antibody fragments" comprise a portion of an intact antibody, generally the antigen binding or variable region of the intact antibody. Examples of antibody fragments include Fab, Fab', F(ab')2, and Fv fragments, diabodies, single chain antibody molecules, and multispecific antibodies formed from antibody fragments.

The term "variable" is used herein to describe certain portions of the variable domains which differ in sequence among antibodies and are used in the binding and specificity of each particular antibody for its particular antigen. However, the variability is not usually evenly distributed through the variable domains of antibodies. It is typically concentrated in three segments called complementarity determining regions (CDRs) or hypervariable regions both in the light chain and the heavy chain variable domains. The more highly conserved portions of the variable domains are called the framework (FR). The variable domains of native heavy and light chains each comprise four FR regions, largely adopting a β-sheer configuration, connected by three CDRs, which form loops connecting, and in some cases forming part of, the β-sheet structure. The CDRs in each chain are held together in close proximity by the FR regions and, with the CDRs from the other chain, contribute to the formation of the antigen binding site of antibodies [see Kabat, E.A. et al., Sequences of Proteins of Immunological Interest, National Institutes of Health, Bethesda, MD (1987)]. The constant domains are not involved directly in binding an antibody to an antigen, but exhibit various effector functions, such as participation of the antibody in antibody-dependent cellular toxicity.

The term "monoclonal antibody" as used herein refers to an antibody obtained from a population of substantially homogeneous antibodies, *i.e.*, the individual antibodies comprising the population are identical except for possible naturally-occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen.

The monoclonal antibodies herein include chimeric, hybrid and recombinant antibodies produced by splicing a variable (including hypervariable) domain of an anti-DR4 antibody with a constant domain (*e.g.* "humanized" antibodies), or a light chain with a heavy chain, or a chain from one species with a chain from another species, or fusions with heterologous proteins, regardless of species of origin or immunoglobulin class or subclass designation, as well as antibody fragments (*e.g.*, Fab, F(ab')₂, and Fv), so long as they exhibit the desired biological activity. See, *e.g.* U.S. Pat. No. 4,816,567 and Mage et al., in Monoclonal Antibody Production Techniques and Applications, pp.79-97 (Marcel Dekker, Inc.: New York, 1987).

Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler and Milstein, Nature, 256:495 (1975), or may be made by recombinant DNA methods such as described in U.S. Pat. No. 4,816,567. The "monoclonal antibodies" may also be isolated from phage libraries generated using the techniques described in McCafferty et al., Nature, 348:552-554 (1990), for example.

"Humanized" forms of non-human (*e.g.* murine) antibodies are specific chimeric immunoglobulins, immunoglobulin chains, or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementary determining region (CDR) of the recipient are replaced by residues from a CDR of a non-human species (donor antibody) such as mouse, rat, or rabbit having the desired specificity, affinity, and capacity. In some instances, Fv framework region (FR) residues of the human immunoglobulin are replaced by corresponding non-human residues. Furthermore, the humanized antibody may comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region or domain (Fc), typically that of a human immunoglobulin.

"Biologically active" and "desired biological activity" for the purposes herein mean having the ability to modulate apoptosis (either in an agonistic or stimulating manner or in an antagonistic or blocking manner) in at least one type of mammalian cell *in vivo* or *ex vivo*.

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured, for instance, by cell viability assays, FACS analysis or DNA electrophoresis, all of which are known in the art.

The terms "treating," "treatment," and "therapy" as used herein refer to curative therapy, prophylactic therapy, and preventative therapy.

The term "mammal" as used herein refers to any mammal classified as a mammal, including humans, cows, horses, dogs and cats. In a preferred embodiment of the invention, the mammal is a human.

### II. Compositions and Methods of the Invention

### A. DR4 Antibodies

The present invention relates to agonist monoclonal antibodies which (a) specifically bind to a DR4 polypeptide comprising amino acid residues 24-218, 1-218, or 1-468 of Fig. 1 (SEQ ID NO:1), (b) induce apoptosis in at least one type of mammalian cell that expresses DR4 polypeptide and (c) do not bind to DcR1 or DcR2. Other types of antibody are described below by way of reference.

### 1. Polyclonal Antibodies

Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the DR4 polypeptide (or a DR4 ECD) or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation. The mammal can then be bled, and the serum assayed for DR4 antibody titer. If desired, the mammal can be boosted until the antibody titer increases or plateaus.

### 2. Monoclonal Antibodies

The antibodies of the present invention are monoclonal antibodies.

Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the DR4 polypeptide (or a DR4 ECD) or a fusion protein thereof, such as a DR4 ECD-IgG fusion protein. The immunizing agent may alternatively comprise a fragment or portion of DR4 having one or more amino acids that participate in the binding of Apo-2L to DR4. In a preferred embodiment, the immunizing agent comprises an extracellular domain sequence of DR4 fused to an IgG sequence, such as described in Example 1.

Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. An example of such a murine myeloma cell line is P3X63AgU.1 described in Example 2 below. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against DR4. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium or RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

As described in the Examples below, various anti-DR4 monoclonal antibodies have been identified and prepared. Certain of those antibodies, referred to as 4E7.24.3 and 4H6.17.8 herein, have been deposited with ATCC and have been assigned deposit accession nos. HB-12454 and HB-12455, respectively. In one embodiment, the monoclonal antibodies of the invention will have the same biological characteristics as the monoclonal antibodies secreted by the hybridoma cell line(s) deposited under Accession No. HB-12454 or Accession No. HB-12455. The term "biological characteristics" is used to refer to the *in vitro* and/or *in vivo* activities or properties of the monoclonal antibody, such as the ability to specifically bind to DR4 or to block, induce or enhance DR4 activation (or DR4-related activities). As disclosed in the present specification, the monoclonal antibody 4E7.24.3 is characterized as specifically binding to DR4 (and having no binding specificity to Apo-2, DcR1 or DcR2), capable of inducing apoptosis, and not capable of blocking DR4. The monoclonal antibody 4H6.17.8 is characterized as specifically binding to DR4 (and having some cross-reactivity to Apo-2 but not to DcR1 or DcR2), capable of inducing apoptosis, and capable of blocking DR4. Optionally, the monoclonal antibodies of the present invention will bind to the same epitope(s) as the 4E7.24.3 or 4H6.17.8 antibodies disclosed herein. This can be determined by conducting various assays, such as described herein and in the Examples. For instance, to determine whether a monoclonal antibody has the same specificity as the 4E7.24.3 or 4H6.17.8 antibodies specifically disclosed, one can compare its activity in DR4 blocking assays or apoptosis induction assays, such as those described in the Examples below.

The antibodies of the invention may also comprise monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published 12/22/94 and U.S. Patent No. 4,342,566. Papain digestion of antibodies typically produces two identical antigen binding fragments, called Fab fragments, each with a single antigen binding site, and a residual Fc fragment. Pepsin treatment yields an F(ab')₂ fragment that has two antigen combining sites and is still capable of cross-linking antigen.

The Fab fragments produced in the antibody digestion also contain the constant domains of the light chain and the first constant domain (CH₁) of the heavy chain. Fab' fragments differ from Fab fragments by the addition of a few residues at the carboxy terminus of the heavy chain CH₁ domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

Single chain Fv fragments may also be produced, such as described in Iliades et al., FEBS Letters, 409:437-441 (1997). Coupling of such single chain fragments using various linkers is described in Kortt et al., Protein Engineering, 10:423-433 (1997).

In addition to the antibodies described above, it is contemplated that chimeric or hybrid antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate.

### 3. Humanized Antibodies

The DR4 antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important in order to reduce antigenicity. According to the "best-fit" method, the sequence of the variable domain of a rodent antibody is screened against the entire library of known human variable domain sequences. The human sequence which is closest to that of the rodent is then accepted as the human framework (FR) for the humanized antibody [Sims et al., J. Immunol., 151:2296-2308 (1993); Chothia and Lesk, J. Mol. Biol., 196:901-917 (1987)]. Another method uses a particular framework derived from the consensus sequence of all human antibodies of a particular subgroup of light or heavy chains. The same framework may be used for several different humanized antibodies [Carter et al., Proc. Natl. Acad. Sci. USA, 89:4285-4289 (1992); Presta et al., J. Immunol., 151:2623-2632 (1993)].

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three dimensional models of the parental and humanized sequences. Three dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequence so that the desired antibody characteristic, such as increased affinity for the target antigen(s), is achieved. In general, the CDR residues are directly and most substantially involved in influencing antigen binding [see, WO 94/04679 published 3 March 1994].

Transgenic animals (e.g., mice) that are capable, upon immunization, of producing a full repertoire of human antibodies in the absence of endogenous immunoglobulin production can be employed. For example, it has been described that the homozygous deletion of the antibody heavy chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge [see, e.g., Jakobovits et al., Proc. Natl. Acad. Sci. USA, 90:2551-2555 (1993); Jakobovits et al., Nature, 362:255-258 (1993); Bruggemann et al., Year in Immuno., 7:33-40 (1993)]. Human antibodies can also be produced in phage display libraries [Hoogenboom and Winter, J. Mol. Biol., 227:381-388 (1991); Marks et al., J. Mol. Biol., 222:581-597 (1991)]. The techniques of Cole et al. and Boemer et al. are also available for the preparation of human monoclonal antibodies (Cole et al., Monoclonal Antibodies and Cancer Therapy, Alan R. Liss, p. 77-96 (1985) and Boerner et al., J. Immunol., 147(1):86-95 (1991)].

### 4. Bispecific Antibodies

Bispecific antibodies are monoclonal, preferably human or humanized, antibodies that have binding specificities for at least two different antigens. In the present case, one of the binding specificities is for the DR4, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

### 5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 6. Triabodies

Triabodies are also within the scope of the invention. Such antibodies are described for instance in Iliades et al., supra and Kortt et al., supra.

### B. Uses for DR4 Antibodies

The DR4 antibodies of the invention have various utilities. For example, DR4 agonistic antibodies may be employed in methods for treating pathological conditions such as malignancies. Diagnosis of such conditions are within the routine skill of the medical practitioner or clinician. In the methods, the DR4 agonistic antibody is administered to a mammal, alone or in combination with still other therapeutic agents or techniques.

The antibody is preferably administered to the mammal in a carrier; preferably a pharmaceutically-acceptable carrier. Suitable carriers and their formulations are described in Remington's Pharmaceutical Sciences, 16th ed., 1980, Mack Publishing Co., edited by Oslo et al. Typically, an appropriate amount of a pharmaceutically-acceptable salt is used in the formulation to render the formulation isotonic. Examples of the carrier include saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of antibody being administered.

The antibody can be administered to the mammal by injection (e.g., intravenous, intraperitoneal, subcutaneous, intramuscular, intraportal), or by other methods such as infusion that ensure its delivery to the bloodstream in an effective form. The antibody may also be administered by isolated perfusion techniques, such as isolated tissue perfusion, to exert local therapeutic effects. Local or intravenous injection is preferred.

Effective dosages and schedules for administering the antibody may be determined empirically, and making such determinations is within the skill in the art. Those skilled in the art will understand that the dosage of antibody that must be administered will vary depending on, for example, the mammal which will receive the antibody, the route of administration, the particular type of antibody used and other drugs being administered to the mammal. Guidance in selecting appropriate doses for antibody is found in the literature on therapeutic uses of antibodies, e.g., Handbook of Monoclonal Antibodies, Ferrone et al., eds., Noges Publications, Park Ridge, N.J., (1985) ch. 22 and pp. 303-357; Smith et al., Antibodies in Human Diagnosis and Therapy, Haber et al., eds., Raven Press, New York (1977) pp. 365-389. A typical daily dosage of the antibody used alone might range from about 1 µg/kg to up to 100 mg/kg of body weight or more per day, depending on the factors mentioned above.

The antibody may also be administered to the mammal in combination with effective amounts of one or more other therapeutic agents. The one or more other therapeutic agents or therapies may include, but are not limited to, chemotherapy, radiation therapy, immunoadjuvants, and cytokines. Other agents known to induce apoptosis in mammalian cells may also be employed, and such agents include TNF-alpha, TNF-beta, CD30 ligand, 4-1BB ligand and Apo-2 ligand.

Chemotherapies contemplated by the invention include chemical substances or drugs which are known in the art and are commercially available, such as Doxorubicin, 5-Fluorouracil, etoposide, camptothecin, Leucovorin, Cytosine arabinoside, Cyclophosphamide, Thiotepa, Busulfan, Cytoxin, Taxol, Methotrexate, Cisplatin, Melphalan, Vinblastine and Carboplatin. Preparation and dosing schedules for such chemotherapy may be used according to manufacturer's instructions or as determined empirically by the skilled practitioner. Preparation and dosing schedules for such chemotherapy are also described in Chemotherapy Service Ed., M.C. Perry, Williams & Wilkins, Baltimore, MD (1992).

The chemotherapy is preferably administered in a pharmaceutically-acceptable carrier, such as those described above. The mode of administration of the chemotherapy may be the same as employed for the DR4 antibody or it may be administered to the mammal via a different mode. For example, the DR4 antibody may be injected while the chemotherapy is administered orally to the mammal.

Radiation therapy can be administered to the mammal according to protocols commonly employed in the art and known to the skilled artisan. Such therapy may include cesium, iridium, iodine or cobalt radiation. The radiation therapy may be whole body radiation, or may be directed locally to a specific site or tissue in or on the body. Typically, radiation therapy is administered in pulses over a period of time from about 1 to about 2 weeks. The radiation therapy may, however, be administered over longer periods of time. Optionally, the radiation therapy may be administered as a single dose or as multiple, sequential doses.

The antibody may be administered sequentially or concurrently with the one or more other therapeutic agents. The amounts of antibody and therapeutic agent depend, for example, on what type of drugs are used, the pathological condition being treated, and the scheduling and routes of administration but would generally be less than if each were used individually.

Following administration of antibody to the mammal, the mammal's physiological condition can be monitored in various ways well known to the skilled practitioner.

It is contemplated that the blocking DR4 antibodies may also be used in therapy. For example, a blocking DR4 antibody could be administered to a mammal (such as described above) to block receptor binding to Apo-2L, thus increasing the bioavailability of Apo-2L to induce apoptosis.

In another embodiment , methods for employing the antibody in diagnostic assays are provided. For instance, the antibodies may be employed in diagnostic assays to detect overexpression of DR4 in specific cells and tissues. Various diagnostic assay techniques known in the art may be used, such as *in vivo* imaging assays, *in vitro* competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The agonists used in the diagnostic assays can be labeled with a detectable moiety. The detectable moiety should be capable of producing, either directly or indirectly, a detectable signal. For example, the detectable moiety may be a radioisotope, such as ³H, ¹⁴C, ³²P, ^{3s}S, or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al., Nature, 144:945 (1962); David et al., Biochemistry, 13:1014-1021 (1974); Pain et al., J. Immunol. Meth., 40:219-230 (1981); and Nygren, J. Histochem. and Cytochem., 30:407-412 (1982).

DR4 antibodies also are useful for the affinity purification of DR4 from recombinant cell culture or natural sources. In this process, the antibodies against DR4 are immobilized on a suitable support, such a Sephadex resin or filter paper, using methods well known in the art. The immobilized antibody then is contacted with a sample containing the DR4 to be purified, and thereafter the support is washed with a suitable solvent that will remove substantially all the material in the sample except the DR4, which is bound to the immobilized antibody. Finally, the support is washed with another suitable solvent that will release the DR4 from the antibody.

In a further embodiment, there are provided articles of manufacture and kits containing materials useful for treating pathological conditions or detecting or purifying DR4. The article of manufacture comprises a container with a label. Suitable containers include, for example, bottles, vials, and test tubes. The containers may be formed from a variety of materials such as glass or plastic. The container holds a composition having an active agent which is effective for treating pathological conditions or for detecting or purifying DR4. The active agent in the composition is a DR4 antibody and preferably, comprises monoclonal antibodies specific for DR4. The label on the container indicates that the composition is used for treating pathological conditions or detecting or purifying DR4, and may also indicate directions for either *in vivo* or *in vitro* use, such as those described above.

The kit comprises the container described above and a second container comprising a buffer. It may further include other materials desirable from a commercial and user standpoint, including other buffers, diluents, filters, needles, syringes, and package inserts with instructions for use.

The invention will be more fully understood by reference to the following examples. They should not, however, be construed as limiting the scope of the invention.

The following examples are offered for illustrative purposes only, and are not intended to limit the scope of the present invention in any way.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, Virginia.

### EXAMPLE 1

### Expression of DR4 ECD as an Immunoadhesin

A soluble DR4 ECD immunoadhesin construct was prepared. A mature DR4 ECD sequence (amino acids 1-218 shown in Fig. 1) was cloned into a pCMV-1 Flag vector (Kodak) downstream of the Flag signal sequence and fused to the CH1, hinge and Fc region of human immunoglobulin G₁ heavy chain as described previously [Aruffo et al., Cell, 61:1303-1313 (1990)]. The immunoadhesin was expressed by transient transfection into human 293 cells and purified from cell supernatants by protein A affinity chromatography, as described by Ashkenazi et al., supra.

### EXAMPLE 2

### Preparation of Monoclonal Antibodies Specific for DR4

Balb/c mice (obtained from Charles River Laboratories) were immunized by injecting 0.5 µg/50 µl of a DR4 ECD immunoadhesin protein (as described in Example 1 above)(diluted in MPL-TDM adjuvant purchased from Ribi Immunochemical Research Inc., Hamilton, MT) 11 times into each hind foot pad at 3-4 day intervals.

Three days after the final boost, popliteal lymph nodes were removed from the mice and a single cell suspension was prepared in DMEM media (obtained from Biowhitakker Corp.) supplemented with 1% penicillin-streptomycin. The lymph node cells were then fused with murine myeloma cells P3X63AgU.1 (ATCC CRL 1597) using 35% polyethylene glycol and cultured in 96-well culture plates. Hybridomas resulting from the fusion were selected in HAT medium. Ten days after the fusion, hybridoma culture supernatants were screened in an ELISA to test for the presence of monoclonal antibodies binding to the DR4 ECD immunoadhesin protein (described in Example 1).

In the ELISA, 96-well microtiter plates (Maxisorb; Nunc, Kamstrup, Denmark) were coated by adding 50 µl of 2 µg/ml goat anti-human IgG Fc (purchased from Cappel Laboratories) in PBS to each well and incubating at 4°C overnight. The plates were then washed three times with wash buffer (PBS containing 0.05% Tween 20). The wells in the microtiter plates were then blocked with 50 µl of 2.0% bovine serum albumin in PBS and incubated at room temperature for 1 hour. The plates were then washed again three times with wash buffer.

After the washing step, 50 µl of 0.4 µg/ml DR4 ECD immunoadhesin protein in assay buffer was added to each well. The plates were incubated for 1 hour at room temperature on a shaker apparatus, followed by washing three times with wash buffer.

Following the wash steps, 100 µl of the hybridoma supernatants or Protein G-sepharose column purified antibody (10 µg/ml) was added to designated wells. 100 µl of P3X63AgU.1 myeloma cell conditioned medium was added to other designated wells as controls. The plates were incubated at room temperature for 1 hour on a shaker apparatus and then washed three times with wash buffer.

Next, 50 µl HRP-conjugated goat anti-mouse IgG Fc (purchased from Cappel Laboratories), diluted 1:1000 in assay buffer (0.5% bovine serum albumin, 0.05% Tween-20 in PBS), was added to each well and the plates incubated for 1 hour at room temperature on a shaker apparatus. The plates were washed three times with wash buffer, followed by addition of 50 µl of substrate (TMB Microwell Peroxidase Substrate; Kirkegaard & Perry, Gaithersburg, MD) to each well and incubation at room temperature for 10 minutes. The reaction was stopped by adding 50 µl of TMB 1-Component Stop Solution (Diethyl Glycol; Kirkegaard & Perry) to each well, and absorbance at 450 nm was read in an automated microtiter plate reader.

Hybridoma supernatants initially screened in the ELISA were considered for their ability to bind to DR4-IgG but not to CD4-IgG. The supernatants testing positive in the ELISA were further analyzed by FACS analysis using 9D cells (a human B lymphoid cell line expressing DR4; Genentech, Inc.) and FITC-conjugated goat anti-mouse IgG. For this analysis, 25 µl of cells suspended (at 4 X 10⁶ cells/ml) in cell sorter buffer (PBS containing 1% FCS and 0.02% NaN₃) were added to U-bottom microtiter wells, mixed with 100µl of culture supernatant or purified antibody (10µg/ml) in cell sorter buffer, and incubated for 30 minutes on ice. The cells were then washed and incubated with 100 µl FITC-conjugated goat anti-mouse IgG for 30 minutes at 4°C. Cells were then washed twice, resuspended in 150 µl of cell sorter buffer and then analyzed by FACScan (Becton Dickinson, Mountain View, CA).

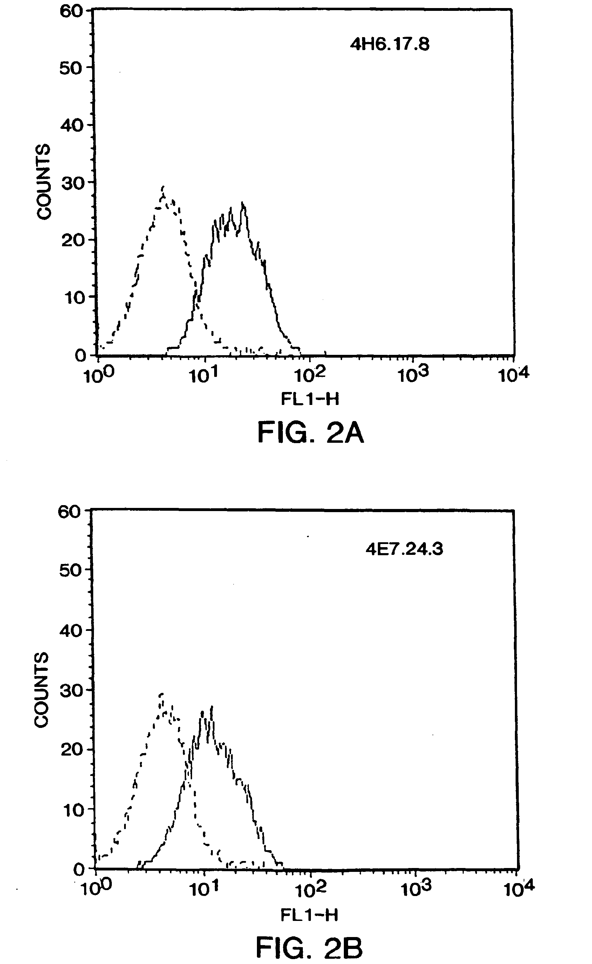

Figure 2 shows the FACS staining of 9D cells. Two particular antibodies, 4E7.24.3 and 4H6.17.8, recognized the DR4 receptor on the 9D cells.

### EXAMPLE 3

### Assay for Ability of DR4 Antibodies to Agonistically induce Apoptosis

Hybridoma supernatants and purified antibodies (as described in Example 2 above) were tested for activity to induce DR4 mediated 9D cell apoptosis. The 9D cells (5 X 10⁵ cells/0.5ml) were incubated with 1 µg of DR4 mAbs (4E7.24.3 or 4H6.17.8; see Example 2 above) or IgG control antibodies in 200 µl complete RPMI media at 4°C for 15 minutes. The cells were then incubated for 5 minutes at 37°C with or without 10 µg of goat anti-mouse IgG Fc antibody (ICN Pharmaceuticals) in 300 µl of complete RPMI. At this point, the cells were incubated overnight at 37°C and in the presence of 7% CO₂. The cells were then harvested and washed once with PBS. The viability of the cells was determined by staining of FITC-annexin V binding to phosphatidylserine according to manufacturer recommendations (Clontech). The cells were washed in PBS and resuspended in 200 µl binding buffer. Ten µl of annexin-V-FITC (1 µg/ml) and 10 µl of propidium iodide were added to the cells. After incubation for 15 minutes in the dark, the 9D cells were analyzed by FACS.

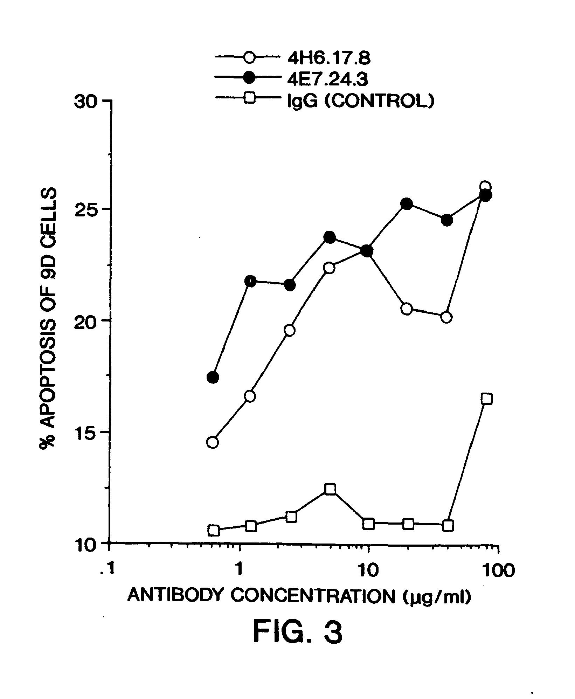

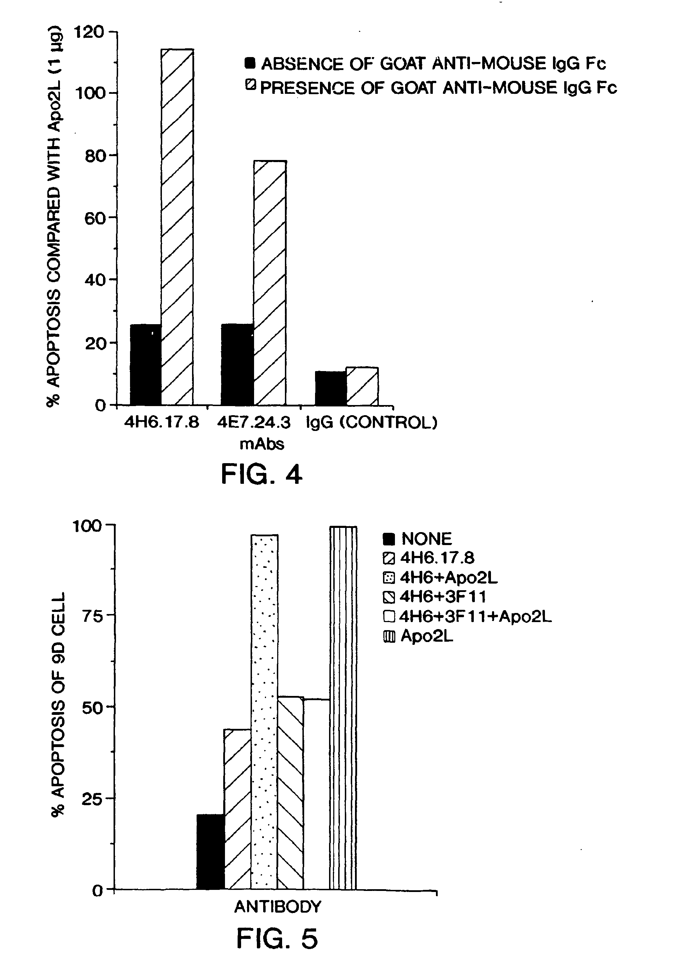

As shown in Figure 3, both DR4 antibodies (in the absence of the goat anti-mouse IgG Fc) induced apoptosis in the 9D cells as compared to the control antibodies. Agonistic activity of both DR4 antibodies, however, was enhanced by DR4 receptor cross-linking in the presence of the goat anti-mouse IgG Fc (See Figure 4). This enhanced apoptosis (Figure 4) by both DR4 antibodies is comparable to the apoptotic activity of Apo-2L in 9D cells (data not shown).

### EXAMPLE 4

### Assay for DR4 Antibody Ability to Block Apo-2L-induced 9D Apoptosis

Hybridoma supernatants and purified antibodies (as described in Example 2 above) were tested for activity to block Apo-2 ligand induced 9D cell apoptosis. The 9D cells (5 X 10⁵ cells/0.5 ml) were suspended in complete RPMI media (RPMI plus 10% FCS, glutamine, nonessential amino acids, penicillin, streptomycin, sodium pyruvate) and placed into individual Falcon 2052 tubes. 0.5 ml of Apo-2L (1 µg/ml; soluble His-tagged Apo-2L prepared as described in WO 97/25428) was suspended into complete RPMI media, preincubated with serially diluted DR4 antibody (4H6.17.8) and/or an Apo-2 antibody (mAb 3F11, Genentech, Inc.), and then added into the tubes containing the 9D cells. The 9D cells were incubated on ice for 15 minutes and then incubated overnight at 37°C and in the presence of 7% CO₂. The incubated cells were then harvested and washed once with PBS. The viability of the cells was determined by staining of FITC-annexin V binding to phosphatidylserine according to manufacturer recommendations (Clontech). Specifically, the cells were washed in PBS and resuspended in 200 µl binding buffer. Ten ml of annexin-V-FITC (1 µg/ml) and 10 µl of propidium iodide were added to the cells. After incubation for 15 minutes in the dark, the 9D cells were analyzed by FACS.

The results are shown in Figure 5. Since 9D cells express more than one receptor for Apo-2L, Apo-2L can induce apoptosis in the 9D cells by interacting with either DR4 or the receptor referred to as Apo-2. Thus, to detect any blocking activity of the DR4 antibodies, the interaction between Apo-2 and Apo-2L needed to be blocked. In combination with the anti-Apo-2 antibody, 3F11, the DR4 antibody 4H6.17.8 was able to block approximately 50% of apoptosis induced by Apo-2L. The remaining approximately 50% apoptotic activity is believed to be due to the agonistic activity of the DR4 antibodies alone, as shown in Figure 5. Accordingly, it is believed that 4H6.17.8 is a blocking DR4 antibody.

### EXAMPLE 5

### Antibody Isotyping

The isotypes of the 4H6.17.8 and 4E7.24.3 antibodies (as described above) were determined by coating microtiter plates with isotype specific goat anti-mouse Ig (Fisher Biotech, Pittsburgh, PA) overnight at 4°C. The plates were then washed with wash buffer (as described in Example 2 above). The wells in the microtiter plates were then blocked with 200 µl of 2% bovine serum albumin and incubated at room temperature for one hour. The plates were washed again three times with wash buffer.

Next, 100 µl of 5 µg/ml of purified DR4 antibodies or 100 µl of the hybridoma culture supernatant was added to designated wells. The plates were incubated at room temperature for 30 minutes and then 50 µl HRP-conjugated goat anti-mouse IgG (as described above) was added to each well. The plates were incubated for 30 minutes at room temperature. The level of HRP bound to the plate was detected using HRP substrate as described above.

The isotyping analysis showed that the 4E7.24.3 and 4H6.17.8 antibodies are IgG1 antibodies.

### EXAMPLE 6

### ELISA Assay to Test Binding of DR4 Antibodies to Other Apo-2L Receptors

An ELISA was conducted to determine if the two DR4 antibodies described in Example 2 were able to bind other known Apo-2L receptors beside DR4. Specifically, the DR4 antibodies were tested for binding to Apo-2 [see, e.g., Sheridan et al., Science, 277:818-821 1 (1997)], DcR1 [Sheridan et al., supra], and DcR2 [Marsters et al., Curr. Biol., al., 7:1003-1006 (1997)]. The ELISA was performed essentially as described in Example 2 above.

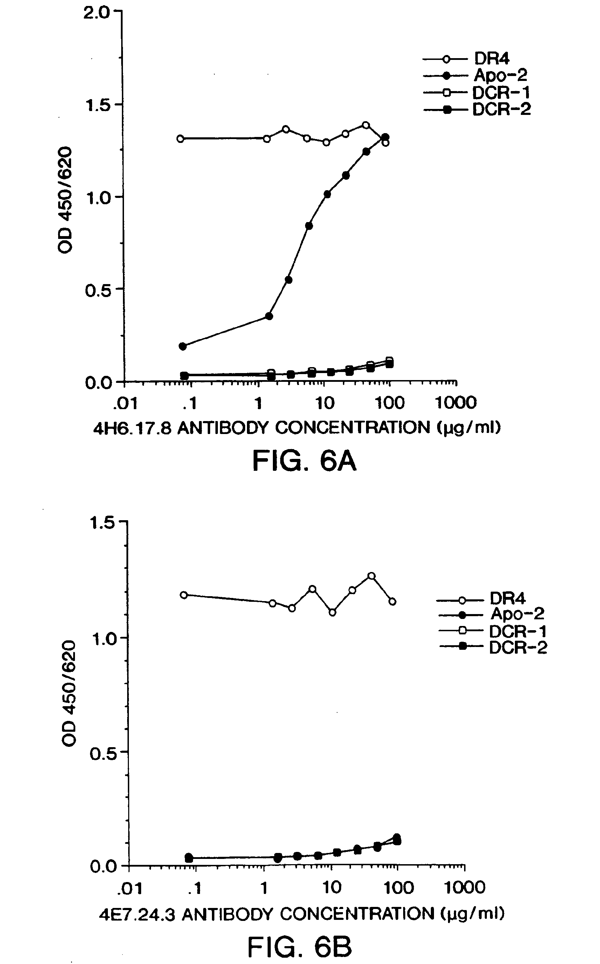

The results are shown in Figure 6. The DR4 antibody 4E7.24.3 bound to DR4, but not to any of the other Apo-2L receptors, Apo-2, DcR1, or DcR2. In contrast, the DR4 antibody 4H6.17.8 showed some cross-reactivity to Apo-2 but not to DcR1 or DcR2.

### Deposit of Material

The following materials have been deposited with the American Type Culture Collection, 10801 University Boulevard, Manassas, Virginia, USA (ATCC):

| Material | ATCC Dep. No. | Deposit Date |
|---|---|---|
| 4E7.24.3 | HB-12454 | Jan. 13, 1998 |
| 4H6.17.8 | HB-12455 | Jan. 13, 1998 |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC '122 and the Commissioner's rules pursuant thereto (including 37 CFR '1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the apperided claims.

## Claims

1. An isolated agonist monoclonal antibody which (a) specifically binds to a Death Receptor 4 (DR4) polypeptide comprising amino acid residues 24-218, 1-218, or 1-468 of Fig. 1 (SEQ ID NO:1), (b) induces apoptosis in at least one type of mammalian cell that expresses DR4 polypeptide and (c) does not bind to decoy receptors DcR1 or DcR2.

2. The antibody of claim 1 which is amman antibody.

3. The antibody of claim 1 which is a chimeric antibody.

4. The antibody of claim 1 which is a humanized antibody.

5. The antibody of claim 1 wherein said mammalian cell is a cancer cell.

6. The antibody of claim 1 wherein the antibody binds to the same epitope as (1) the epitope to which the monoclonal antibody produced by the hybridoma cell line deposited under American Type Culture Collection Accession Number ATCC HB-12454 binds or (2) the epitope to which the monoclonal antibody produced by the hybridoma cell line deposited under the American Type Culture Collection Accession Number ATCC HB-12455 binds.

7. A hybridoma cell line which produces the antibody of claim 1 or claim 6.

8. The hybridoma cell line of claim 7, which has been deposited under American Type Culture Collection Accession Number ATCC HB-12454 or ATCC HB 12455.

9. A monoclonal antibody according to claim 1, produced by a hybridoma cell line of claim 8.

10. An isolated nucleic acid encoding the DR4 antibody of any one of claims 1 to 6.

11. A composition comprising the antibody of any one of claims 1 to 6 and a carrier.

12. The composition of claim 11 wherein said carrier is a pharmaceutically acceptable carrier.

13. A method of inducing apoptosis in mammalian cells in vitro or ex vivo comprising exposing mammalian cells to an effective amount of an antibody according to any of claims 1 to 6.

14. The method of claim 13 wherein said mammalian cells are cancer cells.

15. Use of an antibody according to any one of claims 1 to 6 in the manufacture of a medicament for inducing apoptosis in mammalian cells.

16. The use according to claim 15 wherein said mammalian cells are cancer cells.

17. An article of manufacture, comprising a container and a composition contained within said container, wherein the composition includes an antibody according to any one of claims 1 to 6.

18. A dimeric molecule comprising an antibody according to any one of claims 1 to 6 linked to a heterologous immunoglobulin.

## Patentansprüche

1. Isolierter monoklonaler Agonistenantikörper, der (a) an ein Todesrezeptor-4-(DR4-) Polypeptid, das die Aminosäurereste 24 bis 218, 1 bis 218 oder 1 bis 468 von Fig. 1 (Seq.-ID Nr. 1) umfasst, spezifisch bindet, (b) Apoptose in zumindest einem Typ Säugetierzelle induziert, die DR4-Polypeptid exprimiert, und (c) an die Köderrezeptoren DcR1 und DcR2 nicht bindet.

2. Antikörper nach Anspruch 1, der ein menschlicher Antikörper ist.

3. Antikörper nach Anspruch 1, der ein chimärer Antikörper ist.

4. Antikörper nach Anspruch 1, der ein humanisierter Antikörper ist.

5. Antikörper nach Anspruch 1, worin die Säugetierzelle eine Krebszelle ist.

6. Antikörper nach Anspruch 1, worin der Antikörper an das gleiche Epitop bindet wie (1) das Epitop, an das der monoklonale Antikörper bindet, der von der unter der Zugangsnummer der American Type Culture Collection ATCC HB-12454 hinterlegten Hybridomzelllinie produziert wird, oder wie (2) das Epitop, an das der monoklonale Antikörper bindet, der von der unter der Zugangsnummer der American Type Culture Collection ATCC HB-12455 hinterlegten Hybridomzelllinie produziert wird.

7. Hybridomzelllinie, die den Antikörper nach Anspruch 1 oder Anspruch 6 produziert.

8. Hybridomzelllinie nach Anspruch 7, die unter der Zugangsnummer der American Type Culture Collection ATCC HB-12454 oder ATCC HB-12455 hinterlegt wurde.

9. Monoklonaler Antikörper nach Anspruch 1, produziert von einer Hybridomzelllinie nach Anspruch 8.

10. Isolierte Nucleinsäure, kodierend für den DR4-Antikörper nach einem der Ansprüche 1 bis 6.

11. Zusammensetzung, umfassend den Antikörper 1 bis 6 und einen Träger.

12. Zusammensetzung nach Anspruch 11, worin es sich bei dem Träger um einen pharmazeutisch annehmbaren Träger handelt.

13. Verfahren zur Induktion von Apoptose in Säugetierzellen in vitro oder ex vivo, umfassend das Aussetzen von Säugetierzellen gegenüber einer wirksamen Menge einer Antikörpers nach einem der Ansprüche 1 bis 6.

14. Verfahren nach Anspruch 13, worin es sich bei den Säugetierzellen um Krebszellen handelt.

15. Verwendung eines Antikörpers nach einem der Ansprüche 1 bis 6 zur Herstellung eines Medikaments zur Induktion von Apoptose in Säugetierzellen.

16. Verwendung nach Anspruch 15, worin es sich bei den Säugetierzellen um Krebszellen handelt.

17. Fertigartikel, umfassend einen Behälter und eine in dem Behälter enthaltene Zusammensetzung, worin die Zusammensetzung einen Antikörper nach einem der Ansprüche 1 bis 6 umfasst.

18. Dimeres Molekül, umfassend einen Antikörper nach einem der Ansprüche 1 bis 6, der an ein heterologes Immunglobulin gebunden ist.

## Revendications

1. Anticorps monoclonal agoniste isolé qui (a) se lie spécifiquement à un polypeptide récepteur de mortalité 4 (DR4) comprenant les résidus d'aminoacides 24-218, 1-218 ou 1-468 de la figure 1 (SEQ ID NO:1), (b) induit une apoptose dans au moins un type de cellule de mammifère qui exprime le polypeptide DR4 et (c) de ne se lie pas aux récepteurs leurre DcR1 ou DcR2.

2. Anticorps suivant la revendication 1, qui est un anticorps humain.

3. Anticorps suivant la revendication 1, qui est un anticorps chimère.

4. Anticorps suivant la revendication 1, qui est un anticorps humanisé.

5. Anticorps suivant la revendication 1, ladite cellule de mammifère étant une cellule cancéreuse.

6. Anticorps suivant la revendication 1, ledit anticorps se liant au même épitope que (1) l'épitope auquel l'anticorps monoclonal produit par la lignée cellulaire d'hybridome déposée sous le numéro de dépôt de l'American Type Culture Collection ATCC HB-12454 se lie ou (2) l'épitope auquel l'anticorps monoclonal produit par la lignée cellulaire d'hybridome déposée sous le numéro de dépôt de l'American Type Culture Collection ATCC HB-12455 se lie.

7. Lignée cellulaire d'hybridome qui produit l'anticorps de la revendication 1 ou de la revendication 6.

8. Lignée cellulaire d'hybridome suivant la revendication 7, qui a été déposée sous le numéro de dépôt de l'American Type Culture Collection ATCC HB-12454 ou ATCC HB 12455.

9. Anticorps monoclonal suivant la revendication 1, produit par une lignée cellulaire d'hybridome de la revendication 8.

10. Acide nucléique isolé codant pour l'anticorps DR4 de l'une quelconque des revendications 1 à 6.

11. Composition comprenant l'anticorps de l'une quelconque des revendications 1 à 6 et un support.

12. Composition suivant la revendication 11, dans laquelle ledit support est un support pharmaceutiquement acceptable.

13. Méthode pour induire une apoptose dans des cellules de mammifère in vitro ou ex vivo comprenant l'exposition de cellules de mammifère à une quantité efficace d'un anticorps suivant l'une quelconque des revendications 1 à 6.

14. Méthode suivant la revendication 13, dans laquelle lesdites cellules de mammifère sont des cellules cancéreuses.

15. Utilisation d'un anticorps suivant l'une quelconque des revendications 1 à 6 dans la production d'un médicament pour l'induction d'une apoptose dans des cellules de mammifère.

16. Utilisation suivant la revendication 15, dans laquelle lesdites cellules de mammifère sont des cellules cancéreuses.

17. Article manufacturé, comprenant un récipient et une composition logée dans ledit récipient, dans lequel la composition comprend un anticorps suivant l'une quelconque des revendications 1 à 6.

18. Molécule dimère comprenant un anticorps suivant l'une quelconque des revendications 1 à 6 lié à une immunoglobuline hétérologue.
